# DEMANDE DE BREVET EUROPEEN

(11) **EP 1 938 865 A1**
(43) Date de publication de la demande: **02.07.2008**
(21) Numéro de dépôt: 07123841.4
(22) Date de dépôt: 20.12.2007
(51) Int. Cl.: A61Q 5/06, A61Q 5/12, A61K 8/895, A61K 8/89

(54) **Procédé de traitement des cheveux par une silicone réactive vinylique capable de réagir par hydrosilylation**

(30) Priorité: 20.12.2006 FR 0655754
(71) Demandeur: L'Oréal, 75008 Paris (FR)
(72) Inventeur: Giroud, Franck, 73390 Chamoux sur Gelon (FR)
(74) Mandataire: Fevrier, Murielle Françoise E.

(57) **Abrégé**

La présente invention a pour objet l'utilisation sur les cheveux d'une composition comprenant au moins un composé X et au moins un composé Y, l'un au moins des composé X et Y étant un composé siliconé, lesdits composés X et Y étant *susceptibles de* réagir ensemble par une réaction d'hydrosilylation, pour l'obtention d'un traitement rémanent aux shampooings.
L'invention permet d'obtenir des cheveux particulièrement brillant et doux de façon rémanente, après plusieurs shampoings.

## Description

La présente invention a pour objet un procédé de traitement des cheveux à partir d'une silicone vinylique capable de réagir par hydrosilylation sur les cheveux.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et/ou les teintures. Il en résulte que les cheveux sont souvent difficiles à discipliner, en particulier ils sont difficiles à démêler ou à coiffer, et les chevelures, même abondantes, conservent difficilement une coiffure de bon aspect en raison du fait que les cheveux manquent de vigueur, de volume et de nervosité.

Ainsi, pour remédier à cela, il est maintenant usuel d'utiliser des produits de coiffage qui permettent de conditionner les cheveux en leur apportant notamment du corps, de la masse, de la brillance ou du volume. Ces produits de coiffage sont généralement des compositions cosmétiques capillaires comprenant un ou plusieurs polymères qui présentent une affinité pour les cheveux et qui ont le plus souvent pour fonction de former un film à leur surface en vue de modifier leurs propriétés superficielles, notamment pour les conditionner. Par obtenir un tel effet, il est connu en particulier d'utiliser des polysiloxanes, notamment ceux décrits dans les documents FR 2 799 955, FR 2 799 956, FR 2 799 970 et FR 2 799 971.

Un inconvénient lié à l'utilisation de ces compositions capillaires réside dans le fait que les effets cosmétiques conférés par de telles compositions ont tendance à disparaître, notamment dès le premier shampooing.

Afin de remédier à cet inconvénient, il est envisageable d'accroître la rémanence du dépôt de polymères en effectuant directement une polymérisation de certains monomères au niveau des cheveux. Le document US 4 344 763 décrit une composition de fixation des cheveux à partir d'une silicone réactive aminoalkylalcoxysilane et un titanate d'ester. Il est aussi connu d'effectuer des gainages des cheveux à partir d'une composition comprenant un monomère électrophile de type cyanoacrylate, notamment dans les demandes de brevet FR 2 833 489. Une telle composition permet d'obtenir des cheveux parfaitement gainés et non gras. Cependant, le gainage obtenu ne donne pas une totale satisfaction face aux agents extérieurs tels que le lavage et la transpiration. Par ailleurs le gainage obtenu est sensible aux corps gras tels que le sébum.

Les documents WO01/96450, GB 2 407 496 et EP 465 744 décrivent l'utilisation de silicones réactives particulières pour la réalisation de film sur la peau. Le document WO 01/96450 et GB 2 407 496 décrivent une formulation en une partie qui comprend un polysiloxane ayant des groupes terminaux trialkoxyalkylsilyle un catalyseur, un solvant et éventuellement un alkoxysilane et des charges. Ces compositions permettent d'obtenir par condensation un film sur la peau. Le document EP 465 744 décrit l'utilisation de polysiloxane à groupements aliphatiques insaturés pour réaliser des dispositifs médicaux à usage topique.

Le but de la présente invention est de développer un nouveau procédé de traitement des cheveux qui permet d'obtenir un maintien durable des cheveux tout en conservant de bonnes propriétés cosmétiques.

Ainsi, l'invention a pour objet l'utilisation sur les cheveux d'une composition comprenant au moins un composé X et au moins un composé Y, l'un au moins des composé X et Y étant un composé siliconé, lesdits composés X et Y étant *susceptibles de* réagir ensemble par une réaction d'hydrosilylation, pour l'obtention d'un traitement rémanent aux shampooings.

L'invention permet d'obtenir in situ un gainage homogène lisse et possédant une excellente adhésion sur cheveux, gainage qui est rémanent aux shampoings. Par ailleurs, on a constaté de façon surprenante que les cheveux restaient parfaitement individualisés, pouvaient être coiffés sans problème et que les propriétés de coiffant apportées aux cheveux étaient rémanentes aux shampooings.

### Composés X et Y

Par composé siliconé, on entend un composé comprenant au moins deux unités organosiloxanes. Selon un mode de réalisation particulier, les composés X et les composés Y sont siliconés. Les composés X et Y peuvent être aminés ou non aminés. Ils peuvent comprendre des groupes polaires pouvant être choisis parmi les groupes suivants -COOH, -COO⁻ , -COO- , -OH , - NH₂ , -NH-,-NR-, -SO₃H -SO₃⁻, -OCH₂CH₂-, -O-CH₂CH₂CH₂-, -O-CH₂CH(CH₃)-, -NR₃⁺, -SH, -NO₂, I, Cl, Br, -CN, -PO₄ ³⁻, -CONH- , -CONR-, -CONH₂, -CSNH-, - SO₂-, -SO-, -SO₂NH-, -NHCO- , -NHSO₂-, -NHCOO-, -OCONH-, -NHCSO- et - OCSNH-, R représantant un groupe alkyle.

Selon un autre mode de réalisation, au moins un des composés X et Y est un polymère dont la chaîne principale est formée majoritairement d'unités organosiloxanes.

Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites "room temperature vulcanization".

Les composés X et Y peuvent réagir ensemble à une température variant entre la température ambiante et 180 °C. Avantageusement, les composés X et Y sont susceptibles de réagir ensemble à température ambiante (20 ± 5 °C) et pression atmosphérique, avantageusement en présence d'un catalyseur, par une réaction d'hydrosilylation ou une réaction de condensation, ou une réaction de réticulation en présence d'un peroxyde.

### 1- Composés X et Y susceptibles de réagir par hydrosilylation

Selon un mode de réalisation, les composés X et Y sont susceptibles de réagir ensemble par hydrosilylation, cette réaction pouvant être de manière simplifiée schématisée comme suit : 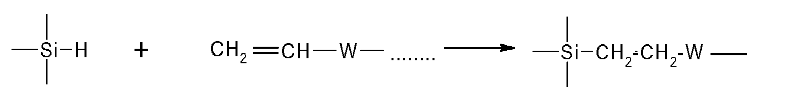 avec W représentant une chaîne carbonée et/ou siliconée contenant un ou plusieurs groupements aliphatiques insaturés.

Dans ce cas, le composé X peut être choisi parmi les composés siliconés comprenant au moins deux groupements aliphatiques insaturés. A titre d'exemple, le composé X peut comprendre une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.

Selon un mode de réalisation, le composé X est choisi parmi les polyorganosiloxanes comprenant au moins deux groupements aliphatiques insaturés, par exemple deux ou trois groupements vinyliques ou allyliques, liés chacun à un atome de silicium.

Selon un mode de réalisation avantageux, le composé X est choisi parmi les polyorganosiloxanes comprenant des unités siloxanes de formule : 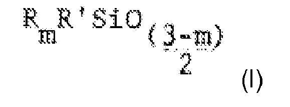 dans laquelle :
- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle ;
- m est égal à 1 ou 2 ; et
- R' représente :
   - un groupement hydrocarboné aliphatique insaturé comprenant de 2 à 10, de préférence de 2 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
   - un groupement hydrocarboné cyclique insaturé comprenant de 5 à 8 atomes de carbone comme par exemple un groupe cyclohexényle.

De préférence R' est un groupement hydrocarboné aliphatique insaturé, de préférence un groupe vinyle.

Selon un mode de réalisation particulier, le polyorganosiloxane comprend également des unités de formule : 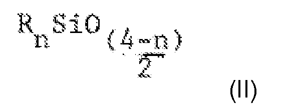 dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé B par hydrosilylation. On peut citer par exemple les résines de type MQ ou MT portant elle-même des extrémités réactives insaturées -CH=CH₂.

Ces résines sont des polymères d'organosiloxanes réticulés.

La nomenclature des résines de silicone est connue sous le nom de "MDTQ", la résine étant décrite en fonction des différentes unités monomériques siloxane qu'elle comprend, chacune des lettres "MDTQ" caractérisant un type d'unité.

La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène

La lettre T représente une unité trifonctionnelle de formule (CH₃)SiO_{3/2} .

Dans les motifs M, D, T définis précédemment, au moins un des groupes méthyle peut être substitué par un groupe R différent du groupe méthyle tel qu'un radical hydrocarboné (notamment alkyle) ayant de 2 à 10 atomes de carbone ou un groupe phényle ou bien encore un groupe hydroxyle.

Enfin, la lettre Q signifie une unité tétrafonctionnelle SiO_{4/2} dans laquelle l'atome de silicium est lié à quatre atomes d'hydrogène eux mêmes liés au reste du polymère. Comme exemples de telles résine, on peut citer les résines de silicone MT telles que les poly(phenyl-vinylsilsesquioxane) comme celle commercialisées sous la référence SST-3PV1 par la société Gelest.

De préférence, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (I) et éventuellement (II) décrites précédemment.

Le composé Y comprend de préférence au moins deux groupes Si-H (groupes hydrogénosilanes) libres.

Le composé Y peut être avantageusement choisi parmi les organosiloxanes comprenant au moins une unité alkylhydrogènosiloxane de formule suivante : 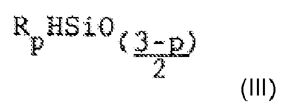 dans laquelle :
R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y organosiloxanes à unités alkylhydrogènosiloxanes peuvent comprendre en outre des unités de formule : 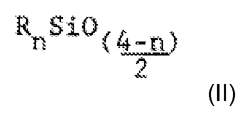 telles que définies plus haut.

Le composé Y peut être une résine de silicone comprenant au moins un motif choisis parmi les motifs M, D, T, Q tels que définis ci-dessus et comprenant au moins un groupe Si-H telles que les poly(methylhydridosilsesquioxane) commercialisées sous la référence SST-3MH1.1 par la société Gelest.

De préférence, ces composés Y organosiloxanes comprennent de 0,5 à 2,5 % en poids de groupes Si-H.

Avantageusement, les radicaux R représentent un groupement méthyle dans les formules (I), (II), (III) ci-dessus.

De préférence, ces organosiloxanes Y comprennent des groupes terminaux de formule (CH₃)₃SiO_{1/2}.

Avantageusement, les organosiloxanes Y comprennent au moins deux unités alkylhydrogènosiloxane de formule (H₃C)(H)SiO et comprennent éventuellement des unités (H₃C)₂SiO.

De tels composés Y organosiloxanes à groupements hydrogénosilane sont décrits par exemple dans le document EP 0465744.

Selon une variante, le composé X est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique / silicone, lesdits oligomères ou polymères portant au moins 2 groupements aliphatiques insaturés réactifs, le composé Y étant choisi parmi les hydrogénosiloxanes cités ci-dessus.

Le composé X, de nature organique, peut alors être choisi parmi les polymères ou oligomères vinyliques, (méth)acryliques, les polyesters, les polyuréthanes et / ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :
a) les polyesters à insaturation(s) éthylénique(s) :

Il s'agit d'un groupe de polymères de type polyester présentant au
   moins 2 doubles liaisons éthyléniques, réparties de manière aléatoire dans la chaîne principale du polymère. Ces polyesters insaturés sont obtenus par polycondensation d'un mélange :
      - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
      - d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.
b) les polyesters à groupes (méth)acrylate latéraux et / ou terminaux:
   Il s'agit d'un groupe de polymères de type polyester obtenus par polycondensation d'un mélange :
      - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
      - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
      - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
   De tels polyesters sont commercialisés par exemple par la société UCB sous les dénominations EBECRYL^{®} (EBECRYL^{®} 450 : masse molaire 1600, en moyenne 6 fonctions acrylate par molécule, EBECRYL^{®} 652 : masse molaire 1500, en moyenne 6 fonctions acrylate par molécule, EBECRYL^{®} 800 : masse molaire 780, en moyenne 4 fonctions acrylate par molécule, EBECRYL^{®} 810 : masse molaire 1000, en moyenne 4 fonctions acrylate par molécule, EBECRYL^{®} 50 000 : masse molaire 1500, en moyenne 6 fonctions acrylate par molécule).
c) les polyuréthannes et / ou polyurées à groupes (méth)acrylate, obtenus par polycondensation :
   - de diisocyanates, triisocyanates et / ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule : 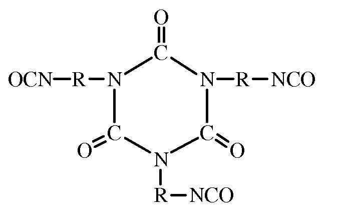 résultant de la trimérisation de 3 molécules de diisocyanates OCN-R-CNO, où R est un radical hydrocarboné linéaire, ramifié ou cyclique comportant de 2 à 30 atomes de carbone ;
   - de polyols, notamment de diols, exempts d'insaturations éthyléniques polymérisables, tels que le 1,4-butanediol, l'éthylèneglycol ou le triméthylolpropane, et / ou de polyamines, notamment de diamines, aliphatiques, cycloaliphatiques et / ou aromatiques ayant notamment de 3 à 50 atomes de carbone, telles que l'éthylènediamine ou l'hexaméthylènediamine, et
   - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   De tels polyuréthannes / polyurées à groupes acrylates sont commercialisés par exemple sous la dénomination SR 368 (tris(2-hydroxyéthyl)isocyanurate-triacrylate) ou CRAYNOR^{®} 435 par la société CRAY VALLEY, ou sous la dénomination EBECRYL^{®} par la société UCB (EBECRYL^{®} 210 : masse molaire 1500, 2 fonctions acrylate par molécule, EBECRYL^{®} 230 : masse molaire 5000, 2 fonctions acrylate par molécule, EBECRYL^{®} 270: masse molaire 1500, 2 fonctions acrylate par molécule, EBECRYL^{®} 8402 masse molaire 1000, 2 fonctions acrylate par molécule, EBECRYL^{®} 8804 masse molaire 1300, 2 fonctions acrylate par molécule, EBECRYL^{®} 220 : masse molaire 1000, 6 fonctions acrylate par molécule, EBECRYL^{®} 2220 : masse molaire 1200, 6 fonctions acrylate par molécule, EBECRYL^{®} 1290 : masse molaire 1000, 6 fonctions acrylate par molécule, EBECRYL^{®} 800 : masse molaire 800, 6 fonctions acrylate par molécule).
   On peut également citer les polyuréthannes aliphatiques diacrylate hydrosolubles commercialisés sous les dénominations EBECRYL^{®} 2000, EBECRYL^{®} 2001 et EBECRYL^{®} 2002, et les polyuréthannes diacrylate en dispersion aqueuse commercialisés sous les dénominations commerciales IRR^{®} 390, IRR^{®} 400, IRR^{®} 422 IRR^{®} 424 par la société UCB.
d) les polyéthers à groupes (méth)acrylate obtenus par estérification, par l'acide (méth)acrylique, des groupes hydroxyle terminaux d'homopolymères ou de copolymères d'alkylèneglycols en C₁-C₄, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.
   Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL^{®} 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL^{®} 160 par la société UCB. Des triacrylates de triméthylolpropane polypropoxylé sont commercialisés par exemple sous les dénominations SR 492 et SR 501 par la société CRAY VALLEY.
e) les époxyacrylates obtenus par réaction entre :
   - au moins un diépoxyde choisi par exemple parmi :
      (i) l'éther diglycidylique de bisphénol A,
      (ii) une résine diépoxy résultant de la réaction entre l'éther diglycidylique de bisphénol A et l'épichlorhydrine,
      (iii) une résine époxyester à extrémités α,ω-diépoxy résultant de la condensation d'un diacide carboxylique ayant de 3 à 50 atomes de carbone avec un excès stoechiométrique de (i) et / ou (ii),
      (iv) une résine époxyéther à extrémités α,ω-diépoxy résultant de la condensation d'un diol ayant de 3 à 50 atomes de carbone avec un excès stoechiométrique de (i) et / ou (ii),
      (v) les huiles naturelles ou synthétiques portant au moins 2 groupes époxyde, telles que l'huile de soja époxydée, l'huile de lin époxydée et l'huile de vernonia époxydée,
      (vi) un polycondensat phénol-formaldéhyde (résine Novolac^{®}), dont les extrémités et/ou les groupes latéraux ont été époxydés,
         et
   - un ou plusieurs acides carboxyliques ou polyacides carboxyliques comportant au moins une double liaison éthylénique en α,β du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.
   De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL^{®} 600 et EBECRYL^{®} 609, EBECRYL^{®} 150, EBECRYL^{®} 860, EBECRYL^{®} 3702 par la société UCB et sous les dénominations PHOTOMER^{®} 3005 et PHOTOMER^{®} 3082 par la société HENKEL.
f) les poly(méth)acrylates d'(alkyle en C₁₋₅₀), ledit alkyle étant linéaire, ramifié ou cyclique, comportant au moins deux fonctions à double liaison éthylénique portées par les chaînes hydrocarbonées latérales et/ou terminales.
   De tels copolymères sont commercialisés par exemple sous les dénominations IRR^{®} 375, OTA^{®} 480 et EBECRYL^{®}2047 par la société UCB.
g) les polyoléfines telles que le polybutène, le polyisobutylène,
h) les perfluoropolyéthers à groupes acrylate obtenus par estérification, par exemple par l'acide (méth)acrylique, de perfluoropolyéthers portant des groupes hydroxyle latéraux et / ou terminaux.
   De tels perfluoropolyéthers α,ω-diols sont décrits notamment dans EP-A-1057849 et sont commercialisés par la société AUSIMONT sous la dénomination FOMBLIN^{®} Z DIOL.
i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.

Les dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST^{®} par la société DENDRITECH.

Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754).

La société PERSTORP commercialise sous la dénomination BOLTORN^{®} des polyesters hyperramifiés. On trouvera sous la dénomination COMBURST^{®} de la société DENDRITECH des polyéthylèneamines hyperramifiées. Des poly(esteramide) hyperramifiés à extrémités hydroxyle sont commercialisés par la société DSM sous la dénomination HYBRANE^{®}.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.

On peut donc utiliser ces polymères dendritiques et hyperramifiés en association avec un ou plusieurs des polymères et/ou oligomères a) à h) ci-dessus.

### 1a Composés réactifs additionnels

Selon un mode de réalisation, la composition conforme à l'invention peut comprendre en outre un composé réactif additionnel tel que :
- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetramethyltetravinylsiloxane,
- des composés silazanes tels que l'hexaméthyldisilazane.

### 1b Catalyseur

La réaction d'hydrosilylation se fait avantageusement en présence d'un catalyseur qui peut être présent dans la composition conforme à l'invention, le catalyseur étant de préférence à base de platine ou d'étain.

On peut citer par exemple les catalyseurs à base de platine déposé sur un support de gel de silice ou de poudre de charcoal (charbon), le chlorure de platine, les sels de platine et d'acides chloroplatiniques.

On utilise de préférence les acides chloroplatiniques sous forme hexahydrate ou anhydre, facilement dispersible dans les milieux organosiliconés.

On peut également citer les complexes de platine tels que ceux à base d'acide chloroplatinique hexahydrate et de divinyl tétraméthyldisiloxane.

Le catalyseur peut être présent dans la composition conforme à la présente invention en une teneur allant 0,0001 % à 20 % en poids par rapport au poids total de la composition.

On peut également introduire dans la composition de l'invention des inhibiteurs ou retardateurs de polymérisation, et plus particulièrement des inhibiteurs du catalyseur, ceci afin d'accroître la stabilité de la composition dans le temps ou de retarder la polymérisation. De façon non limitative, on peut citer les polyméthylvinylsiloxanes cycliques, et en particulier le tétravinyl tétraméthyl cyclotétrasiloxane, les alcools acétyléniques, de préférence volatiles, tels que le méthylisobutynol.

La présence de sels ioniques, tels que l'acétate de sodium, dans la composition peut avoir une influence dans la vitesse de polymérisation des composés.

De façon avantageuse, les composés X et Y sont choisis parmi les composés siliconés susceptibles de réagir par hydrosilylation ; en particulier le composé X est choisi parmi les polyorganosiloxanes comprenant des unités de formule (I) décrits ci-dessus et le composé Y est choisi parmi les organosiloxanes comprenant des unités alkylhydrogènosiloxanes de formule (III) décrits ci-dessus.

Selon un mode de réalisation particulier, le composé X est un polydiméthylsiloxane à groupements vinyliques terminaux, et le composé Y est le méthylhydrogénosiloxane.

A titre d'exemple d'une combinaison de composés X et Y réagissant par hydrosilylation, on peut citer les références suivantes proposée par la société Dow Corning : DC 7-9800 Soft Skin Adhesive Parts A & B, ainsi que les mélanges A et B suivants préparés par Dow Corning:

### MELANGE A :

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

### MELANGE B:

| Ingrédient (Nom INCI) | N°CAS | Teneurs (%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

Selon un mode de réalisation, la réaction d'hydrosilylation entre les composés X et Y est accélérée par un apport de chaleur en élevant par exemple la température du système entre 25 °C et 180 °C. Le système réagira notamment sur les fibres kératiniques.

D'une façon générale, les composés X et Y réagissent ensemble, le pourcentage molaire de X par rapport à l'ensemble des composés X et Y, c'est-à-dire le ratio X / (X+Y) x 100, peut varier de 5 % à 95 %, de préférence de 10 % à 90 %, mieux encore de 20 % à 80 %.

De même, le pourcentage molaire de Y par rapport à l'ensemble des composés X et Y, c'est-à-dire le ratio Y / (X+Y) x 100, peut varier de 5 % à 95 %, de préférence de 10 % à 90 %, mieux encore de 20 % à 80 %.

Le composé X peut présenter une masse moléculaire moyenne en poids (Mw) allant de 150 à 1 000 000, de préférence de 200 à 800 000, de préférence encore de 200 à 250 000.

Le composé Y peut présenter une masse moléculaire moyenne en poids (Mw) allant de 200 à 1 000 000, de préférence de 300 à 800 000, de préférence encore de 500 à 250 000.

Le composé X peut représenter de 0,5 % à 95 % en poids par rapport au poids total de la composition, de préférence de 1 % à 90 % et mieux de 5 % à 80%.

Le composé Y peut représenter de 0,05 % à 95 % en poids par rapport au poids total de la composition, de préférence de 0,1 % à 90 % et mieux de 0,2 % à 80 %.

Le ratio entre les composés X et Y, lorsque X et Y sont différents peut être varié de manière à moduler la vitesse de réaction et donc la vitesse de formation du film ou encore de manière à adapter les propriétés du film formé (par exemple ses propriétés adhésives) selon l'application recherchée.

En particulier, les composés X et Y peuvent être présents en un ratio X / Y molaire allant de 0,05 à 20 et mieux de 0,1 à 10.

Selon une variante, le procédé de l'invention comprend deux étapes, une première étape qui consiste à appliquer sur les cheveux au moins une composition comprenant le composé X et une seconde étape qui consiste à appliquer au moins une composition comprenant le composé Y, les étapes pouvant être inversées.

Lorsque le catalyseur est présent, il peut être soit dans la composition contenant X soit dans la composition comprenant Y, soit dans les deux compositions, soit dans une troisième composition, l'ordre d'application des trois compositions pouvant être quelconque.

Selon une autre variante, le procédé de l'invention comprend une première étape qui consiste à appliquer une composition comprenant X et Y, et une seconde composition comprenant le catalyseur.

Selon le procédé de l'invention, il est possible d'appliquer sur les cheveux plusieurs fois en alternance une composition comprenant X et une composition comprenant Y.

Le procédé de la présente invention peut comprendre une ou plusieurs étapes supplémentaires telles que l'application d'un polymère filmogène, un polymère fixant, un agent de conditionnement, une étape de lavage, de séchage.

La composition de l'invention peut contenir de l'eau ou un ou plusieurs solvants organiques, ou un mélange d'eau et d'un ou plusieurs solvants organiques.

Par solvant organique, on entend une substance organique liquide à la température de 25°C à pression atmosphérique (760mm de Hg) capable de dissoudre une autre substance sans la modifier chimiquement.

Le ou les solvants organiques utiles dans la présente invention sont distincts des composés X et Y définis précédemment.

Le solvant organique est par exemple choisi parmi les alcools aromatiques tels que l'alcool benzylique ; les alcools gras liquides , notamment en C10-C30; les polyols modifiés ou non tels que le glycérol, le glycol, le propylène glycol, le dipropylène glycol, le butylène glycol, le butyle diglycol ; les silicones volatiles telles que les silicones linéaires à chaîne courte l'héxaméthyldisiloxane, l'octométhyltrisiloxane, les silicones cycliques, tels que l'octaméthyl cyclotetrasiloxane, la décaméthylcyclopentasiloxane, la dodecaméthylcyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de C5 à C10; les acides gras liquides, les esters gras liquides et plus particulièrement les benzoates ou les salicylates d'alcool gras liquides.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en C5-C10, l'acétone, la méthyléthylcétone, les esters d'acides en C1-C20 liquides et d'alcools en C1-C8 tels que l'acétate de méthyle, l'acétate de butyle, l'acétate d'éthyle et le myristate d'isopropyle, le diméthoxyéthane, le diéthoxyéthane, les alcools gras liquides en C10-C30 tels que l'alcool oléique, les esters d'alcools gras ou d'acide gras tels que les benzoates d'alcool gras en C10-C30 et leurs mélanges ; l'isononanoate d'isononyle, le malate d'isostéaryle, le tétra-isostéarate de pentaérythrityle, le trimélate de tridécyle ; l'huile de polybutène ; le mélange cyclopentasiloxane (14,7% en poids)/polydiméthylsiloxane dihydroxylé en positions α et γ (85,3% en poids), ou leurs mélanges.

Selon un mode de réalisation préféré, le solvant organique est constitué par une silicone ou un mélange de silicone tels que les polydiméthylsiloxanes liquides et les polydiméthylsiloxanes modifiées liquides, la viscosité de la silicone et/ou du mélange de silicone à 25°C est comprise entre 0.1 cst et 1 000 000cst et plus préférentiellement entre 1 cst et 30 000cst.

On citera de préférence les huiles et mélanges d'huiles suivantes :
- le mélange de polydiméthylsiloxane alpha-omega-dihydroxylé/cyclopentadiméthylsiloxane (14,7/85,3) commercialisé par Dow Corning sous le nom de DC 1501 Fluid,
- le mélange de polydiméthylsiloxane alpha-omega-dihydroxylé/polydiméthylsiloxane commercialisé par Dow Corning sous le nom de DC 1503 Fluid,
- le mélange de diméthicone /cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;
   et les mélanges respectifs de ces huiles.

Le ou les solvants organiques et l'eau lorsqu'elle est présente représente généralement de 0,01 à 99 %, de préférence de 50 à 99 % en poids par rapport au poids total de la composition.

La composition de l'invention peut contenir, outre le ou les solvants organiques, de l'eau dans des proportions variant de 1 à 99 %, de préférence de 1 à 50 % par rapport au poids total de la composition. Cependant, selon un mode de réalisation particulier, la composition de l'invention est anhydre c'est-à-dire contenant moins de 1% en poids d'eau par rapport au poids total de la composition.

La composition de l'invention peut aussi se présenter sous forme d'une émulsion et/ou être encapsulée. Lorsque la composition est une émulsion, elle est par exemple constituée par une phase dispersée ou continue qui peut être de l'eau, des alcools aliphatiques en C1-C4 ou leurs mélanges et une phase organique insoluble dans l'eau.

La composition conforme à l'invention peut également contenir outre les composés X et Y, au moins un agent utilisé habituellement en cosmétique choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des parfums, des conservateurs, des tensio-actifs anioniques, cationiques, non ioniques ou amphotères, des polymères fixants ou non, des protéines, des vitamines, des colorants directs autres que les colorants hydrophobes de l'invention, des colorants d'oxydation, des agents nacrants, des propulseurs, et des épaississants minéraux ou organiques tels que le benzylidène-sorbitol et les N-acylaminoacides, les cires oxyéthylénées ou non, les paraffines, les acides gras solides en C₁₀-C₃₀ tels que l'acide stéarique, l'acide laurique, les amides gras ou d'acides gras solides.

Les compositions peuvent se présenter sous différentes formes galéniques tels qu'une lotion, une mousse aérosol, un après shampooing ou un shampooing, un gel, une cire. Les compositions peuvent être contenues dans un flacon pompe, un spray aérosol. Les compositions de l'invention après application sur la chevelure peuvent être rincées ou non.

Lorsque la composition est contenue dans un aérosol, elle peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non (butane, propane, isobutane) et leurs mélanges. On pourra le cas échéant utiliser des aérosols à poche(s) contenant une ou plusieurs poches.

L'invention est illustrée plus en détails par les exemples décrits ci-après. Sauf indication contraire, les quantités indiquées sont exprimées en pourcentage massique.

### Exemples

Les compositions suivantes ont été préparées à partir d'un mélange de POLYDIMETHYLHYDROGENOSILOXANE et de POLYDIMETHYLSILOXANE DIVINYLE, avec un catalyseur à base de platine commercialisé par DOW CORNING sous la dénomination 7-9800 PART A et 7-9800 PART B.

Dans 90 grammes d'éthanol sont introduits 5 grammes de DOW CORNING 7-9800 PART A et 5 grammes de DOW CORNING 7-9800 PART B. Les deux composés siliconés sont ensuite émulsifiés dans l'éthanol à l'aide d'une turbine de type TURAX.
10 grammes de la précédente émulsion sont appliqués sur une demi tête constituée par des cheveux caucasiens naturels mi longs et 10 grammes d'éthanol sont appliqués sur l'autre demi tête. La chevelure ainsi traitée est laissée une nuit à température ambiante.

L'ensemble de la tête est ensuite lavé avec un shampoing doux.

Après 5 shampooings, on note que la partie de la chevelure traitée suivant le procédé de la présente invention est plus brillante, se coiffe plus facilement et les cheveux sont plus doux et plus corporisés que la partie de la chevelure traitée uniquement à l'éthanol.

En conclusion, l'application d'une silicone réactive de l'invention apporte des effets cosmétiques bénéfiques et ceci de façon rémanente aux shampooings.

## Revendications

1. Utilisation sur les cheveux d'une composition comprenant au moins un composé X et au moins un composé Y, l'un au moins des composé X et Y étant un composé siliconé, lesdits composés X et Y étant *susceptibles de* réagir ensemble par une réaction d'hydrosilylation, pour l'obtention d'un traitement rémanent aux shampooings.

2. Utilisation selon la revendication 1 en présence d'au moins un catalyseur.

3. Utilisation selon la revendication 2 dans laquelle le catalyseur est un catalyseur au platine ou à l'étain.

4. Utilisation selon la revendication 3, **caractérisée en ce que** le composé X est choisi parmi les composés siliconés comprenant au moins deux groupements aliphatiques insaturés.

5. Utilisation selon la revendication 4 dans laquelle le composé X est un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal).

6. Utilisation selon la revendication 5, **caractérisée en ce que** le composé X est porteur d'au moins un groupe polaire.

7. Utilisation selon l'une des revendications 4 à 6, **caractérisée en ce que** le composé X est choisi parmi les polyorganosiloxanes comprenant des unités siloxanes de formule : 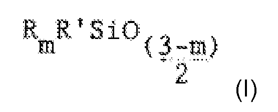 dans laquelle :
- R représente un groupe hydrocarboné monovalent, linéaire ou cyclique, comprenant de 1 à 30 atomes de carbone,
- m est égal à 1 ou 2 et
- R' représente :
• un groupement hydrocarboné aliphatique insaturé comprenant de 2 à 10, ; ou
• un groupement hydrocarboné cyclique insaturé comprenant de 5 à 8 atomes de carbone.

8. Utilisation selon la revendication 8 dans laquelle le polyorganosiloxane de formule (I) est tel que R' représente un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène.

9. Utilisation selon la revendication 8 ou 7, **caractérisée en ce que** R représente un radical alkyle comprenant de 1 à 10 atomes de carbone ou encore un groupement phényle et R' est choisi parmi les groupements vinyle.

10. Utilisation selon la revendication 9, **caractérisée en ce que** les polyorganosiloxanes comprennent en outre des unités de formule 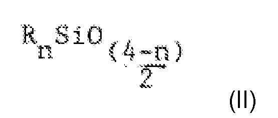 dans laquelle R est un groupe tel que défini dans la revendication 9, est n est égal à 1,2 ou 3.

11. Utilisation selon l'une quelconque des revendications précédentes **caractérisée en ce que** le composé X est choisi parmi les oligomères ou polymères organiques, les oligomères ou polymères hybrides organique/silicone, lesdits oligomères ou polymères portant au moins 2 groupements aliphatiques insaturés réactifs, le composé Y étant choisi parmi les hydrogénosiloxanes tels que définis précédemment.

12. Utilisation selon l'une des revendications 3 à 11, **caractérisée en ce que** le composé Y est choisi parmi les organosiloxanes comprenant au moins deux groupes Si-H libres

13. Utilisation selon l'une des revendications 3 à 12, **caractérisée en ce que** le composé Y est choisi parmi les organosiloxanes comprenant au moins une unité alkylhydrogènosiloxanes de formule suivante : 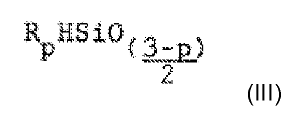 dans laquelle :
R représente un groupe hydrocarboné monovalent, linéaire ou cyclique, comprenant de 1 à 30 atomes de carbone ou un groupe phényle, et p est égal à 1 ou 2.

14. Utilisation selon la revendication 13, dans laquelle le composé Y est tel que les radicaux R représentent un groupement méthyle.

15. Utilisation selon l'une des revendications 13 à 14, dans laquelle les organosiloxanes Y comprennent au moins deux unités alkylhydrogènosiloxane de formule H₃CHSiO et comprennent éventuellement des unités (H₃C)₂SiO

16. Utilisation selon l'une des revendications 1 à 15, **caractérisée en ce que** le catalyseur est un catalyseur à base de platine ou d'étain présent dans l'une ou l'autre des compositions comprenant X et/ou Y ou dans une composition séparée.

17. Utilisation selon la revendication précédente, **caractérisée en ce que** le catalyseur représente de 0,0001% à 20% en poids par rapport au poids total de la composition le comprenant.

18. Utilisation selon l'une des quelconque des revendications 1 à 15, **caractérisée en ce que** le composé X est un polydiméthylsiloxane à groupements vinyliques terminaux et le composé Y est le méthylhydrogénosiloxane.

19. Utilisation selon l'une quelconque des revendications précédentes comprenant en mélange avec le composé X et/ou Y ou séparément au moins une charge choisie parmi la silice ou la silice traitée en surface.

20. Utilisation selon l'une des revendications précédentes, **caractérisée en ce que** le composé X présente une masse moléculaire moyenne en poids (Mw) allant de 150 à 1 000 000, de préférence de 200 à 800 000, de préférence encore de 200 à 250 000.

21. Utilisation selon l'une des revendications précédentes, **caractérisée en ce que** le composé X représente de 0,5% à 95% en poids par rapport au poids total de la composition, de préférence de 1% à 90% et mieux de 5% à 80%.

22. Utilisation selon l'une des revendications précédentes, **caractérisée en ce que** le composé Y représente de 0,001 % à 95% en poids par rapport au poids total de la composition, de préférence de 1% à 90% et mieux de 5% à 80%.

23. Utilisation selon l'une des revendications précédentes, **caractérisée en ce que** les composés X et Y sont présents dans la composition en un ratio molaire X/Y allant de 0,05 à 20 et mieux de 0,1 à 10.

24. Utilisation selon l'une quelconque des revendications précédentes dans lequel les composés X et Y sont appliqués sur les cheveux simultanément.

25. Utilisation selon l'une quelconque des revendications 1 à 24 comprenant deux étapes, une première étape qui consiste à appliquer au moins une composition comprenant le composé X et une seconde étape qui consiste à appliquer au moins une composition comprenant le composé Y, les étapes pouvant être inversées.

26. Utilisation selon l'une quelconque des revendications 2 à 24 qui comprend une première étape qui consiste à appliquer une composition comprenant X et Y, et une seconde composition comprenant au moins un catalyseur.

27. Utilisation selon la revendication 25 qui comprend en outre une troisième étape comprenant l'application d'une composition comprenant au moins un catalyseur, l'ordre d'application des trois compositions étant quelconque.
